# EUROPEAN PATENT APPLICATION

(11) **EP 2 093 224 A1**
(43) Date of publication of application: **26.08.2009**
(21) Application number: 09002814.3
(22) Date of filing: 01.05.2008
(51) Int. Cl.: C07D 403/06, A61K 31/404, A61P 25/06

(54) **Process for preparing eletriptan hydrobromide form beta**

(30) Priority: 01.05.2007 US 927241; 30.07.2007 US 962632; 19.09.2007 US 973603
(62) Divisional of application: 08767568.2
(71) Applicant: Plus Chemicals B.V., 3841 RK Mijdrecht (NL)
(72) Inventor: Mendelovici, Marioara, Rehovot (IL); Lancry, Eli, Modiin (IL); Moshkovits-Kaptsan, Rinat, Raanana (IL)
(74) Representative: Wong, Kit Yee

(57) **Abstract**

The invention encompasses a process for preparing eletriptan hydrobromide Form β.

## Description

### CROSS-REFERENCE TO RELATED APPLICATIONS

This application claims the benefit of U.S. provisional application Nos. 60/927,241 filed May 1, 2007; 60/962,632 filed July 30, 2007; and 60/973,603 filed September 19, 2007, hereby incorporated by reference.

### FIELD OF THE INVENTION

The present invention relates to amorphous eletriptan hydrobromide, processes for preparing it and pharmaceutical compositions of it. The invention also relates to processes for preparing other forms of eletriptan hydrobromide, such as Form α and Form β.

### BACKGROUND OF THE INVENTION

Eletriptan-HBr (ELT-HBr), (R)-5-[2-(Phenylsulfonyl) ethyl]-3-[(1-methyl-2-pyrrolidinyl) methyl]-1H-indol hydrobromide, having the following chemical structure of formula (I): 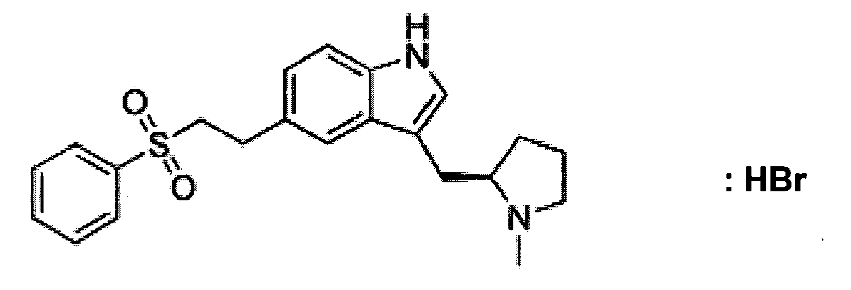 is a selective 5-hydroxytryptamine 1B/1D (5-HT1B/1D) receptor agonist. Eletriptan binds with high affinity to 5-HT1B, 5-HT1D and 5-HT1F receptors, has modest affinity for 5-HT1A, 5-HT1E, 5-HT2B and 5-HT7 receptors, and little or no affinity for 5-HT2A, 5-HT2C, 5-HT3, 5-HT4, 5-HT5A and 5-HT6 receptors. Eletriptan has no significant affinity or pharmacological activity at adrenergic alpha1, alpha2, or beta; dopaminergic D 1 or D2; muscarinic; or opioid receptors.

This pharmaceutical ingredient is commercially available as RELPAX® by Pfizer for the treatment of migraine headaches. It is orally administered as tablets containing 24.2 mg or 48.5 mg of ELT-HBr equivalent to 20 mg or 40 mg of eletriptan.

Eletriptan base and eletriptan succinate are believed to have been described for the first time in U.S. Patent No. 5,545,644 (referred to herein as U.S. Patent '644).

U.S. Patent No. 6,110,940 and its divisional patent, U.S. Patent No. 6,380,226 (herein U.S. patents '940 and '226) describe crystalline form α of eletriptan hydrobromide. Form α is characterized by IR peaks at 3371, 3293, 2713, 2524, 1419, 1343, 1307, 1264, 1151, 1086, 1020, 1008, 999, 922, 900, 805, 758, 740, 728, 689, 672, 652, 640, 598, 581, 573, 531, 498, 465, 457, 443, 428, 422, 414 and 399 cm⁻¹. Form α is further characterized by a PXRD pattern obtained using copper radiation filtered with graphite monochromator (λ= 0.15405 nm), which shows main peaks at 9.7, 10.7, 15.9, 16.5, 17.8, 18.3, 19.3, 19.8, 20.1, 21.2, 24.4, 25.5, 25.8, 26.7, 27.6, and 29.4 degrees 20. U.S. Patent '940 also reports the crystalline form of ELT-HBr designated as form β and preparation thereof. Form β is characterized by IR peaks at 3239, 2672, 2656, 2632, 1409, 1366, 1351, 1334, 1303, 1293, 1152, 1138, 1122, 1098, 1086, 791, 771, 746, 688, 634, 557, 528, 484, 476, 469, 463, 455, 432, 424, 413 and 401 cm⁻¹. Form β is further characterized by a PXRD pattern obtained using copper radiation filtered with graphite monochromator (λ= 0.15405 nm), which shows main peaks at 11.0, 17.2, 19.2, 20.1, 21.6, 22.6, 23.6 and 24.8 degrees 2θ.

Form α is prepared either by treatment of a solution of eletriptan base in acetone with an aqueous solution of hydrogen bromide (49% w/w, 20-25°C) followed by crystallization of the isolated crude oil from 2-propanol, or by recrystallizing Form β from a mixture of acetone and water to which an additional amount of acetone is added to induce precipitation of Form α.

U.S. Patent Publication 2002/013358 (the '358 publication) and parallel PCT application published as WO 00/32589 describe crystalline ELT-HBr monohydrate and the preparation thereof. The monohydrate ELT-HBr is characterized by the following IR peaks:

| | | | | | | | | | |
|---|---|---|---|---|---|---|---|---|---|
| 406.9 | 429.6 | 456.6 | 473.9 | 497.1 | 529.2 | 553.9 | 566.4 | 592.2 | 601.1 |
| 606.2 | 642.2 | 665.0 | 667.3 | 689.1 | 729.5 | 747.8 | 767.2 | 793.0 | 807.2 |
| 822.0 | 841.2 | 852.8 | 870.1 | 876.3 | 890.9 | 926.3 | 937.9 | 948.9 | 970.5 |
| 985.0 | 997.3 | 1010.2 | 1017.4 | 1071.0 | 1085.7 | 1102.4 | 1141.0 | 1150.4 | 1178.5 |
| 1189.1 | 1241.0 | 1267.1 | 1287.8 | 1305.4 | 1328.5 | 1346.7 | 1353.4 | 1387.3 | 1408.8 |
| 1444.9 | 1458.1 | 1482.5 | 1549.0 | 1581.3 | 1611.6 | 1622.0 | 1646.6 | 1703.4 | 1827.7 |
| 1893.3 | 1913.9 | 1937.2 | 1978.6 | 2001.7 | 2676.9 | 2852.6 | 2846.6 | 2893.3 | 2921.4 |
| 2952.9 | 2971.5 | 2994.2 | 3013.8 | 3038.5 | 3054.5 | 3071.0 | 3079.6 | 3117.0 | 3131.2 |
| 3246.0 | 3473.4 | | | | | | | | |

The '358 publication also discloses processes to prepare an anhydrous crystalline form of ELT-HBr that is a mixture of α and β forms.

The present invention relates to the solid-state physical properties of eletriptan hydrobromide. These properties can be influenced by controlling the conditions under which ELT-HBr is obtained in solid form. Solid-state physical properties include, for example, the flow-ability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid-state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid-state form of a compound may also affect its behavior on compaction and its storage stability.

These practical physical characteristics are influenced by the conformation and orientation of molecules in the unit cell, which defmes a particular polymorphic form of a substance. The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetric (DSC) and can be used to distinguish some polymorphic forms from others. A particular polymorphic form may also give rise to distinct spectroscopic properties that may be detectable by powder X-ray crystallography, solid-state ¹³C NMR spectrometry and infrared spectrometry.

One of the most important physical properties of a pharmaceutical compound, which can form polymorphs, is its solubility in aqueous solution, particularly the solubility in gastric juices of a patient. Other important properties relate to the ease of processing the form into pharmaceutical dosages, as the tendency of a powdered or granulated form to flow and the surface properties that determine whether crystals of the form will adhere to each other when compacted into a tablet.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

There is a need in the art for new polymorphs of ELT-HBr and processes for the preparation thereof and also additional processes for preparing existing forms, such as Form α and Form β.

### SUMMARY OF THE INVENTION

One embodiment of the invention is the amorphous form of eletriptan hydrobromide (ELT-HBr).

Another embodiment of the invention encompasses a process for preparing amorphous ELT-HBr comprising slurrying ELT-HBr Form β in a solvent selected from a group consisting of glycerol, mixtures of water and tetrahydrofuran, and mixtures of ethylacetate and water, wherein the mixture of water and THF contains at least about 2% of water by volume.

In another embodiment, the invention encompasses a process for preparing amorphous ELT-HBr comprising crystallizing ELT-HBr from methylisobutyl ketone.

Another embodiment of the invention encompasses a process for preparing amorphous ELT-HBr comprising slurrying wet ELT-HBr monohydrate in a solvent selected from the group consisting of ethanol and mixtures of ethanol and ethylacetate.

Another embodiment of the invention encompasses a process for preparing amorphous ELT-HBr comprising crystallizing ELT-HBr from mixtures of ethylacetate and water or from ethylene glycol.

Another embodiment of the invention encompasses a process for preparing amorphous ELT-HBr comprising spray drying a solution of ELT-HBr in methanol.

One embodiment of the invention encompasses a process for preparing amorphous ELT-HBr comprising heating wet monohydrate ELT-HBr at a temperature of about 20°C to about 80°C at a pressure of about atmospheric pressure to about 0.1 mm Hg.

A further embodiment of the invention encompasses the preparation of ELT-HBr Form α or ELT-HBr Form β or ELT-HBr monohydrate from amorphous ELT-HBr.

Another embodiment of the invention encompasses a process for preparing crystalline ELT-HBr Form α by a process comprising slurrying ELT-HBr Form β in solvent selected from a group consisting of isobutanol, methylacetate, mixtures of THF and water, and cyclohexane, wherein the mixture of water and THF contains less than about 2% to about 0.5% of water by volume.

One embodiment of the invention encompasses a process for preparing crystalline ELT-HBr Form α comprising crystallizing ELT-HBr from ethanol

Another embodiment of the invention encompasses a process for preparing ELT-HBr Form α comprising heating wet crystalline ELT-HBr Form β.

One embodiment of the invention encompasses a process for preparing crystalline ELT-HBr Form β comprising reacting eletriptan base and hydrobromic acid in isopropanol (IPA).

Another embodiment of the invention encompasses a process for preparing ELT-HBr Form β comprising heating wet amorphous ELT-HBr at a temperature of about 60°C.

Another embodiment of the invention encompasses a process for preparing ELT-HBr Form β comprising combining eletriptan p-toluenesulfonic acid ("ELT-PTSA") with water, adding methyl tert-butyl ether ("MTBE") and NH₃ to cause a phase separation where after the organic phase is dried and combined with isopropyl alcohol ("IPA") or mixtures of IPA and acetone, the pH is adjusted by the addition of HBr in IPA and the resulting mixture is cooled and the ELT-HBr Form β is collected.

Yet another embodiment of the invention encompasses a process for preparing ELT-HBr monohydrate comprising heating wet amorphous ELT-HBr at a temperature of about room temperature to about 60°C.

In yet another embodiment the invention encompasses a pharmaceutical composition comprising amorphous ELT-HBr and at least one pharmaceutically acceptable excipient.

One embodiment of the invention encompasses a pharmaceutical composition comprising amorphous ELT-HBr made by the processes of the invention, and at least one pharmaceutically acceptable excipient.

Yet another embodiment of the invention encompasses a process for preparing a pharmaceutical composition of ELT-HBr comprising combining amorphous ELT-HBr with at least one pharmaceutically acceptable excipient.

Another embodiment of the invention encompasses the use of amorphous ELT-HBr for the manufacture of a medicament for the treatment of migraine headaches.

Another embodiment of the invention encompasses the use of amorphous ELT-HBr made by the processes of the invention for the manufacture of a pharmaceutical composition.

### BRIEF DESCRIPTION OF THE FIGURES

Figure 1 illustrates a powder X-ray diffraction pattern of amorphous Eletriptan hydrobromide (ELT-HBr) obtained by slurry and crystallization processes.
Figure 2 illustrates a powder X-ray diffraction pattern of amorphous Eletriptan hydrobromide (ELT-HBr) obtained in examples 11 and 12.
Figure 3 illustrates an X-ray diffraction pattern of amorphous Eletriptan hydrobromide (ELT-HBr) obtained in example 28 (the peak at 28.5 deg. 2θ is due to additional Si powder).

### DETAILED DESCRIPTION OF THE INVENTION

As used herein, unless otherwise defined, the term "room temperature" refers to a temperature range of about 20°C to about 25°C. The term "volume" refers to volume that is measured by grams per millilitres.

As used herein, when referring to ELT-HBr, the term "wet" refers to the first ELT-HBr solid product that was recovered from its reaction mixture and that was not subjected to a drying process, even at room temperature. Typically, such a product contains solvent or a mixture of solvents in an amount of about 5% to about 60% by weight determined by Loss on Drying ("LOD"), wherein the solvent or the mixture of solvents can be an organic solvent, water, and a mixture thereof.

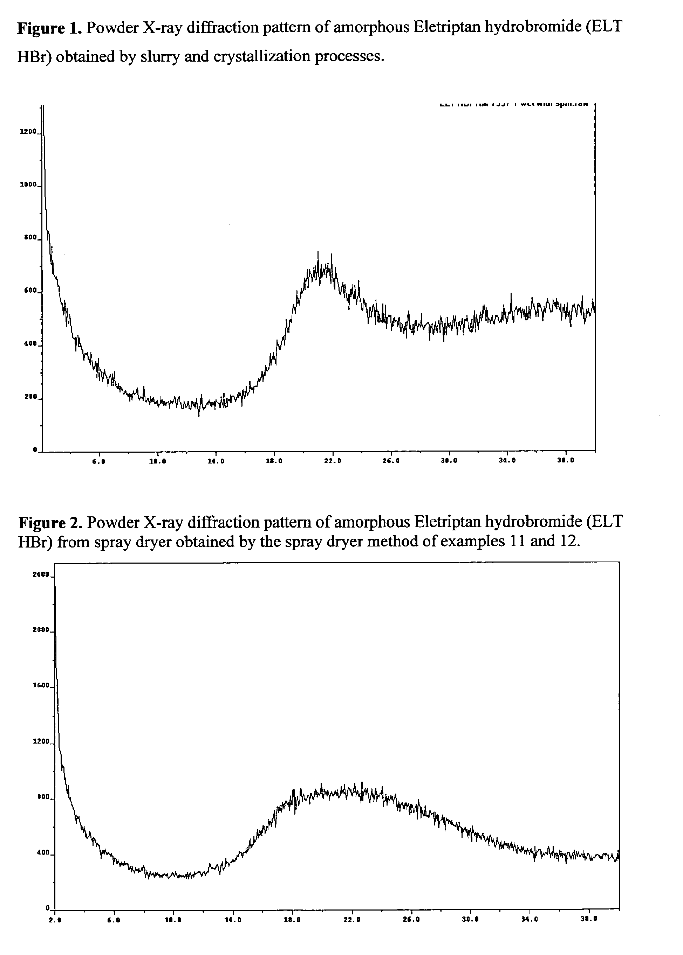

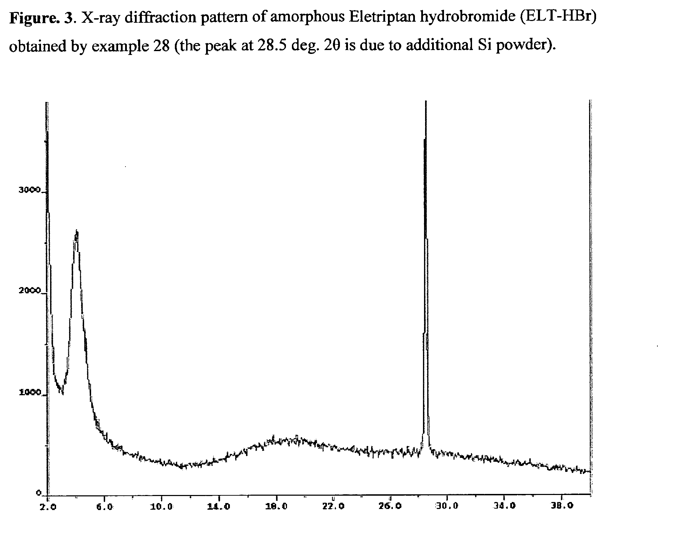

The invention provides amorphous form of ELT-HBr. The amorphous ELT-HBr is characterized by the PXRD patterns as depicted in Figures 1-3.

The above amorphous ELT-HBr has less than about 10% by weight, more preferably less than about 5% by weight, and most preferably less than about 1% by weight of crystalline ELT-HBr. Preferably, the crystalline ELT-HBr is selected from the group consisting of: crystalline ELT-HBr characterized by PXRD pattern having peaks at 9.7, 10.7, 15.9, 16.5, 17.8, 18.3, 19.3, 19.8, 20.1, 21.2, 24.4, 25.5, 25.8, 26.7, 27.6, and 29.4 degrees 2θ, designated Form α ; crystalline ELT-HBr characterized by PXRD pattern having peaks at 11.0, 17.2, 19.2, 20.1, 21.6, 22.6, 23.6 and 24.8 degrees 2θ, designated as Form β; crystalline ELT-HBr characterized by PXRD pattern having peaks at 9.8, 12.5, 13.2, 13.6, 15.1, 16.2, 17.0, 17.5, 18.9, 19.7, 20.0, 21.9, 23.1, 23.6, 24.1, 27.4 and 29.7 degrees 20 designated monohydrate ELT-HBr, and mixtures thereof. The content of crystalline ELT-HBr Form α in amorphous ELT-HBr can be measured by PXRD using any one of the peaks at 9.7, 10.7, 15.9, 16.5, 17.8, 18.3, 19.3, 19.8, 20.1, 21.2, 24.4, 25.5, 25.8, 26.7, 27.6, and 29.4 degrees 20. The content of crystalline ELT-HBr Form β in amorphous ELT-HBr can be measured by PXRD using any one of the peaks at 11.0, 17.2, 19.2, 20.1, 21.6, 22.6, 23.6 and 24.8 degrees 2θ, and the content of monohydrate ELT-HBr in amorphous ELT-HBr can be measured by PXRD using any one of the peaks at 9.8, 12.5, 13.2, 13.6, 15.1, 16.2, 17.0, 17.5, 18.9, 19.7, 20.0, 21.9, 23.1, 23.6, 24.1, 27.4 and 29.7 degrees 2θ.

The above amorphous ELT-HBr can be prepared by a process comprising slurrying, with or without stirring, ELT-HBr Form β in a solvent selected from a group consisting of glycerol, mixtures of water and tetrahydrofuran, and mixtures of ethylacetate and water, wherein the mixture of water and THF contains at least about 2% to about 10% of water by volume.

Preferably, the mixture of water and THF contains at least about 5% to about 10% of water by volume and more preferably about 7% of water to about 10%. Preferably, the solvent is a mixture of water and THF or a mixture of ethylacetate and water.

The starting Form β can be prepared, for example, according to the process disclosed in U.S. Patent No. 6,110,940.

The ratio of ELT-HBr Form β and the solvent in the slurry is about 1:5 mg/mL to about 1:20 mg/mL, and preferably about 1:5 to about 1:10 mg/mL. If a solvent system comprising ethylacetate and water is used, preferably the amount of water in the mixtures is about 2% to about 30% by volume and more preferably about 5% to about 30% by volume.

The temperature in which Form β is slurried in the mixtures of ethylacetate and water, and THF and water is dependent on the kind of solvents in the mixture. When a mixture of ethylacetate and water is used, Form β may be slurried at a temperature of about room temperature to about 80°C, preferably, at about 20°C to about 80°C, more preferably, at about 20°C to about 40°C, and most preferably, at about 20°C. When a mixture of THF and about 2% of water by volume is used, Form β may be slurried at a temperature of about room temperature to about 60°C, and preferably, at about 40°C to about 60°C.

The slurrying may be performed with stirring such that Form β is slurried for a time sufficient to allow the transformation to occur. The total time necessary to allow the transformation to occur will depend on the starting amount of Form β and the solvent used. For example, about 1 hour to 3 hours are required when slurrying about 50mg Form β in ethylacetate and water. In another example, only about 0.5 hours to 1.5 hours is required when slurrying about 50mg Form β in THF and 2% of water by volume. When the solvent is glycerol, the slurrying is done for about 3 hours at room temperature.

The process for preparing amorphous ELT-HBr may further comprise a recovery process. The recovery of amorphous ELT-HBr may be done by a method that doesn't include a drying step, even when carried out at room temperature. Such methods include but aren't limited to cooling the slurry at about room temperature to about -20°C and filtering the slurry.

In another embodiment, the amorphous ELT-HBr can be prepared by a process comprising crystallizing ELT-HBr from methylisobutyl ketone. Typically, the crystallization comprises dissolving ELT-HBr in methylisobutyl ketone to form a solution, and precipitating amorphous ELT-HBr to obtain a suspension. Preferably, the solution is provided by combining ELT-HBr and methylisobutyl ketone, and heating the combination to about 80°C. Preferably, the combination is heated for about 0.5 hours. Preferably, precipitation of amorphous ELT-HBr occurs by cooling the solution at a temperature of about 10°C to obtain a suspension. Typically, to increase the yield of the precipitated amorphous eletriptan hydrobromide, the suspension is heated for about 3 hours.

The process may further comprise a recovery process. The recovery can be done by any method that doesn't include a drying step. Such methods include, but aren't limited to, filtering the slurry.

Amorphous ELT-HBr can further be prepared by a process comprising slurrying wet ELT-HBr monohydrate in a solvent selected from a group consisting of ethanol and mixtures of ethanol and ethylacetate. The starting ELT-HBr monohydrate can be prepared, for example, by the process disclosed in U.S. Patent Publication 2002/013358.

When the solvent is ethanol the slurrying is preferably done at about room temperature to about the reflux temperature of the solvent, and preferably from about 20°C to about 40°C. Depending on the amount of amorphous ELT-HBr to be produced, the slurrying is preferably done for about 1.5 hours to 24 hours and more preferably for about 3 to 5 hours.

When the solvent is a mixture of ethanol and ethylacetate, the slurrying is preferably done at about room temperature to about 80°C, more preferably at about 60°C to 80°C. Depending on the amount of amorphous ELT-HBr to be produced, the slurrying preferably is done for about 0.5 hour to 24 hours and more preferably for about 3 to 5 hours. Preferably, ethanol is present in the above solvent mixture in an amount of about 50% to 80% by volume and more preferably in about 70% to 80% by volume.

Typically, the process for preparing amorphous ELT-HBr can further comprise a recovery process. The recovery can be done by a method that doesn't include a drying step. Such methods include, but are not limited to, cooling the slurry and filtering the slurry.

In another embodiment, the amorphous ELT-HBr can also be prepared by a process comprising crystallizing ELT-HBr from mixtures of ethylacetate and water or from ethylene glycol.

In a preferred embodiment, the solution of ELT-HBr in a mixture of ethylacetate and water is provided by heating a slurry of ELT-HBr in a mixture of solvents.
Preferably, the heating is to a temperature of about 60°C to 80°C, and more preferably about 80°C. Preferably, the heating is performed for about 0.5 hour to 24 hours, and more preferably for about 1.5 hours to 5 hours.

Preferably, the amount of water in the solvent system is about 2% to about 30% by volume, more preferably, about 2% to about 7% by volume, most preferably about 7% by volume.

In another embodiment, the solution of ELT-HBr in ethylene glycol is provided by combining ELT-HBr and ethylene glycol at a temperature of about 0°C to about 25°C, preferably, at about 20°C to about 25°C.

Preferably, the combination is heated for about 0.5 an hour to about 2 hours, and more preferably, for about 0.5 hour to about 2 hours, providing the solution. The ratio of ELT-HBr and solvent in the solution is preferably about 1:5 mg/mL.

Typically, the suspension of amorphous ELT-HBr is provided by cooling the solution. Preferably, the solution is cooled at a temperature of about 10°C to about -20°C more preferably at about -20°C. When the solvent is a mixture of ethylacetate and water, the cooling is conducted at about 10°C to -20°C and preferably about 0°C to 10°C. When the solvent is ethylene glycol, the cooling is conducted at about 0°C to -20°C, and preferably about -5°C to -20°C. Optionally, the obtained suspension is further cooled to increase the yield of the precipitated product. Preferably, the suspension is cooled for about 1 to about 3 hours, more preferably, for about 2 to about 3 hours.

The process for preparing amorphous ELT-HBr may further comprise a recovery process. The recovery of amorphous ELT-HBr may be done by a method that doesn't include a drying step. Such method includes, but is not limited to, filtering the suspension.

The above amorphous ELT-HBr may be prepared by a process comprising spray drying a solution of ELT-HBr in methanol. According to Remington: The Science and Practice of Pharmacy, 19th Ed., vol. II, pg. 1627, spray drying consists of bringing together a highly dispersed liquid and a sufficient volume of hot gas to produce evaporation and drying of the liquid droplets. A typical spray-drying apparatus includes a drying chamber, atomizing means for atomizing a solvent-containing feed into the drying chamber, a source of heated drying gas that flows into the drying chamber to remove solvent from the atomized-solvent-containing feed and product collection means located downstream of the drying chamber. Examples of such apparatus include Niro Models PSD-1, PSD-2 and PSD-4 (Niro A/S, Soeborg, Denmark). Modifications to the spray drying technique are disclosed in WO 03/063821 and WO 03/063,822.

Preferably, the solution is prepared by dissolving ELT-HBr in methanol. Preferably, the solution is spray-dried at an inlet temperature of from about room temperature to about 200°C, more preferably of about 60°C to about 120°C. Preferably, the solution is spray-dried at an outlet temperature of from about room temperature to about 120°C, more preferably of about 37°C to about 75°C.

In another embodiment, amorphous ELT-HBr can be prepared by a process comprising heating wet monohydrate ELT-HBr at a temperature of at least about room temperature to about 80°C and at atmospheric pressure to about 0.1 mm Hg.

Preferably, the wet monohydrate ELT-HBr is heated at a temperature of about room temperature to about 50°C, more preferably at about room temperature. Preferably, the wet monohydrate ELT-HBr is heated at a pressure of about 10 to about 30 mm Hg, and most preferably at about 20 mm Hg.

Typically, the heating step is conducted for a period of time sufficient to allow the transformation of the monohydrate to amorphous form. Preferably, the heating is conducted for about 18 hours to about 5 days, preferably, for about 18 hours to 30 hours.

In a preferred embodiment, amorphous ELT-HBr can be prepared by heating wet monohydrate ELT-HBr at a temperature of at least about room temperature to about 80°C, at a reduced pressure of about 20 mm Hg to about 0.1 mm Hg.. Preferably, the wet monohydrate is heated at a temperature of about 50°C to about 70°C, more preferably at about 50°C. Preferably, the monohydrate is heated at the above conditions for about 18 hours.

The amorphous ELT-HBr of the invention is easy to handle because it filters very quickly.

A further embodiment of the invention encompasses the preparation of ELT-HBr Form α, ELT-HBr Form β, or ELT-HBr monohydrate from amorphous ELT-HBr.

The invention also provides a process for preparing crystalline ELT-HBr Form α by a process comprising slurrying ELT-HBr Form β in a solvent selected from a group consisting of isobutanol, methylacetate, mixtures of THF and water, and cyclohexane, wherein the mixture of water and THF contains from less than about 2% of water by volume to about 0.5% of water by volume. Preferably, the solvent is isobutanol, methylacetate, or cyclohexane. The ratio of ELT-HBr and the solvent in the slurry is preferably, about 1:1 to about 1:10 mg/mL, and more preferably of about 1:5 mg/mL.

The slurrying may be preformed at a temperature of room temperature to about 80°C, with a preferred temperature at about 50°C to about 80°C. The starting Form β is slurried for a time sufficient to allow the transformation to occur. Preferably, it is slurried for a total time of about 2 hours to 25 hours, and more preferably for about 3 to about 23 hours.

The slurrying can optionally be done in one step or step-wise. When done in one step, the slurrying may be conducted at about 40°C to 80°C and preferably at about 60°C to 80°C. Typically, the slurrying is performed for about 2 to about 25 hours, and preferably for about 3 to about 23 hours. When done step-wise, the slurrying may be conducted at about 50°C to 80°C, for about 3 hours, and then at about 15°C to about 25°C, preferably at about 20°C to about 15°C for about 30 minutes to about 2 hours, and more preferably for about 30 minutes to about 1 hour.

The process for preparing crystalline ELT-HBr Form α may further comprise a recovery process. The recovery of crystalline ELT-HBr Form α may be by a process that doesn't include drying at reduced pressure, for example, by filtering the slurry.

Crystalline ELT-HBr Form α can also be prepared by a process comprising crystallizing ELT-HBr from ethanol. Typically, the crystallization is done by a process comprising providing a solution of ELT-HBr in ethanol, and precipitating crystalline ELT-HBr Form α to obtain a suspension. The solution of ELT-HBr in ethanol is provided at about room temperature. Typically, the precipitation is done by cooling the solution. Preferably, the solution is cooled at a temperature of about -5°C to about -20°C, and more preferably at about -19°C. The yield of the precipitated crystalline Form α may optionally be increased by cooling for about 24 hours to about 3 days, preferably 3 days.

The process for preparing crystalline ELT-HBr Form α may further comprise a recovery process. The recovery of crystalline ELT-HBr Form α may be done, for example, by filtering the suspension.

ELT-HBr Form α may also be prepared by a process comprising heating wet crystalline ELT-HBr Form β.

Preferably, the heating is conducted at a temperature of about 45°C to about 60°C, more preferably, of about 50°C to about 55°C. Heating may optionally be done under reduced pressure, preferably at a pressure of about 10 to about 30 mm Hg, and more preferably at about 10 to about 20 mm Hg. Typically, the drying is conducted for a period of time to allow the transformation of the Form to β to Form α. The drying may be conducted for about 16 to about 24 hours.

In one embodiment, crystalline ELT-HBr From β is prepared by reacting eletriptan base and hydrobromic acid in isopropanol (IPA).

Initially, eletriptan base is combined with IPA to obtain a solution. Then, the solution is combined with hydrobromic acid to obtain a reaction mixture. Preferably the hydrobromic acid is in a form of a solution, wherein the solvent is a mixture of water and IPA. Optionally, seeding of crystals of ELT-HBr Form β can be done before the reaction with HBr, to facilitate the crystallization of Form β, thus providing a suspension.

Typically, the reaction with the acid reduces the pH of the reaction mixture. Preferably, the pH of the reaction mixture is about 6 to about 8, more preferably of about 6.5 to about 7.5, and most preferably of about 7.

The suspension may be maintained to increase the yield of the precipitated product. Preferably, the suspension is maintained for about 30 minutes to about 24 hours, preferably for about 2 hours to about 10 hours, more preferably for about 6 hours

The reaction mixture typically comprises ELT-HBr, which is separated from the rest of the reaction mixture, and then combined with another amount of IPA providing a new mixture, which is put in an ultrasonic bath. The new mixture may optionally remain in the ultrasonic bath for about 5 to about 15 minutes, preferably for about 10 minutes. The new mixture may then be cooled in a refrigerator, preferably at a temperature of about 5°C to about 2°C, for about 16 to about 30 hours, preferably for about 16 to about 20 hours, providing the crystalline product.

The process for preparing crystalline ELT-HBr Form β may further comprise a recovery process. The recovery of crystalline ELT-HBr Form β may be done, for example, by filtering and washing. Optionally, these methods can also include drying at a temperature of about 30°C to about 45°C, under a pressure of about 0.1 mmHg to about 20 mm Hg. Preferably, the drying is done at a temperature of about 40°C to about 45°C.

In another embodiment, ELT-HBr Form β can be prepared by a process comprising heating wet amorphous ELT-HBr at a temperature of about 50°C to about 70°C. Preferably, the heating is done at about 60°C. Preferably, the heating is done at a pressure of about 0.1 mmHg to about 20 mmHg, preferably 20 mm Hg.

The invention also provide a process for preparing crystalline ELT-HBr Form β by combining eletriptan p-toluenesulfonic acid (ELT-PTSA) in water to form a solution, and adding methyl tert-butyl ether and NH₃ to adjust the pH, preferably to a pH of about 10.5 to 11.0. The solution is then separated into an organic phase and inorganic phase. The organic phase is combined with a 5% sodium carbonate solution and another phase separation is preformed. The resulting organic phase is then dried. Optionally, IPA can be added and the resulting mixture can be dried a second time. IPA or a mixture of IPA and acetone is then added to the dried organic phase to form a new mixture. The mixture is then optionally heated. A solution of HBr in IPA is added to adjust the pH, preferably to a pH of about 6.6 to about 7.5. The resulting mixture can optionally be seeded with ELT-HBr Form β. The mixture is then cooled and the resulting ELT-HBr Form β is recovered.

In another embodiment, monohydrate ELT-HBr is prepared by a process comprising heating wet amorphous ELT-HBr at a temperature of about room temperature to about 60°C.

In yet another embodiment the invention encompasses a pharmaceutical composition comprising amorphous ELT-HBr and at least one pharmaceutically acceptable excipient.

One embodiment of the invention encompasses a pharmaceutical composition comprising amorphous ELT-HBr made by the processes of the invention, and at least one pharmaceutically acceptable excipient.

Yet another embodiment of the invention encompasses a process for preparing a pharmaceutical composition of ELT-HBr comprising combining amorphous ELT-HBr with at least one pharmaceutically acceptable excipient.

Another embodiment of the invention encompasses the use of amorphous ELT-HBr for the manufacture of a medicament for the treatment of migraine headaches.

Another embodiment of the invention encompasses the use of amorphous ELT-HBr made by the processes of the invention for the manufacture of a pharmaceutical composition.

Methods of administration of a pharmaceutical composition of the invention may comprise administration in various preparations depending on the age, sex, and symptoms of the patient. The pharmaceutical compositions can be administered, for example, as tablets, pills, powders, liquids, suspensions, emulsions, granules, capsules, suppositories, injection preparations (solutions and suspensions), and the like. When the pharmaceutical composition comprises amorphous ELT-HBr the liquid pharmaceutical composition is a suspension or emulsion, wherein amorphous ELT-HBr retains its form.

In addition, pharmaceutical compositions of the present invention can contain inactive ingredients such as diluents, carriers, fillers, bulking agents, binders, disintegrants, disintegration inhibitors, absorption accelerators, wetting agents, lubricants, glidants, surface active agents, flavoring agents, and the like.

Diluents increase the bulk of a solid pharmaceutical composition and can make a pharmaceutical dosage form containing the composition easier for the patient and care giver to handle. Diluents for solid compositions include, for example, microcrystalline cellulose (e.g., Avicel^{®}), microfme cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., Eudragit^{®}), potassium chloride, powdered cellulose, sodium chloride, sorbitol, or talc.

Carriers for use in the pharmaceutical compositions may include, but are not limited to, lactose, white sugar, sodium chloride, glucose, urea, starch, calcium carbonate, kaolin, crystalline cellulose, or silicic acid.

Binders help bind the active ingredient and other excipients together after compression. Binders for solid pharmaceutical compositions include for example acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, or starch.

Disintegrants can increase dissolution. Disintegrants include, for example, alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Disintegration inhibitors may include, but are not limited to, white sugar, stearin, coconut butter, hydrogenated oils, and the like.

Absorption accelerators may include, but are not limited to, quaternary ammonium base, sodium laurylsulfate, and the like.

Wetting agents may include, but are not limited to, glycerin, starch, and the like. Adsorbing agents may include, but are not limited to, starch, lactose, kaolin, bentonite, colloidal silicic acid, and the like.

A lubricant can be added to the composition to reduce adhesion and ease release of the product from a punch or dye during tableting. Lubricants include for example magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Glidants can be added to improve the flowability of non-compacted solid composition and improve the accuracy of dosing. Excipients that can function as glidants include for example colloidal silicon dioxide, magnesium trisilicate, powdered cellulose, starch, talc and tribasic calcium phosphate.

Flavoring agents and flavor enhancers make the dosage form more palatable to the patient. Common flavoring agents and flavor enhancers for pharmaceutical products that can be included in the composition of the present invention include for example maltol, vanillin, ethyl vanillin, menthol, citric acid, fumaric acid, ethyl maltol, and tartaric acid.

Tablets can be further coated with commonly known coating materials such as sugar coated tablets, gelatin film coated tablets, tablets coated with enteric coatings, tablets coated with films, double layered tablets, and multi-layered tablets. Capsules can be coated with shell made, for example, from gelatin and optionally contain a plasticizer such as glycerin and sorbitol, and an opacifying agent or colorant.

Solid and liquid compositions can also be dyed using any pharmaceutically acceptable colorant to improve their appearance and/or facilitate patient identification of the product and unit dosage level.

In liquid pharmaceutical compositions of the present invention, the amorphous ELT-HBr is suspended together with any other solid ingredients, which may be dissolved or suspended, in a liquid carrier, such as water, vegetable oil, alcohol, polyethylene glycol, propylene glycol or glycerin. In suspension the amorphous ELT-HBr retains its crystalline form.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention can also contain viscosity enhancing agents to improve the mouth-feel of the product and/or coat the lining of the gastrointestinal tract. Such agents include for example acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar can be added to improve the taste.

Preservatives and chelating agents such as alcohol, sodium benzoate, butylated hydroxy toluene, butylated hydroxyanisole and ethylenediamine tetraacetic acid can be added at safe levels to improve storage stability.

A liquid pharmaceutical composition according to the present invention can also contain a buffer such as guconic acid, lactic acid, citric acid or acetic acid, sodium guconate, sodium lactate, sodium citrate or sodium acetate.

Selection of excipients and the amounts to use can be readily determined by an experienced formulation scientist in view of standard procedures and reference works known in the art.

A composition for tableting or capsule filing can be prepared by wet granulation. In wet granulation some or all of the active ingredients and excipients in powder form are blended and then further mixed in the presence of a liquid, typically water, which causes the powders to clump up into granules. The granulate is screened and/or milled, dried and then screened and/or milled to the desired particle size. The granulate can then be tableted or other excipients can be added prior to tableting, such as a glidant and/or a lubricant.

A tableting composition can be prepared conventionally by dry blending. For instance, the blended composition of the actives and excipients can be compacted into a slug or a sheet and then comminuted into compacted granules. The compacted granules can be compressed subsequently into a tablet.

As an alternative to dry granulation, a blended composition can be compressed directly into a compacted dosage form using direct compression techniques. Direct compression produces a more uniform tablet without granules. Excipients that are particularly well-suited to direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention can comprise any of the aforementioned blends and granulates that were described with reference to tableting, only they are not subjected to a fmal tableting step.

When shaping the pharmaceutical composition into pill form, any commonly known excipient used in the art can be used. For example, carriers include, but are not limited to, lactose, starch, coconut butter, hardened vegetable oils, kaolin, talc, and the like. Binders used include, but are not limited to, gum arabic powder, tragacanth gum powder, gelatin, ethanol, and the like. Disintegrating agents used include, but are not limited to, agar, laminalia, and the like.

For the purpose of shaping the pharmaceutical composition in the form of suppositories, any commonly known excipient used in the art can be used. For example, excipients include, but are not limited to, polyethylene glycols, coconut butter, higher alcohols, esters of higher alcohols, gelatin, semisynthesized glycerides, and the like.

When preparing injectable pharmaceutical compositions and suspensions they are sterilized and are preferably made isotonic to blood. Injection preparations may use carriers commonly known in the art. For example, carriers for injectable preparations include, but are not limited to, water, ethyl alcohol, propylene glycol, ethoxylated isostearyl alcohol, polyoxylated isostearyl alcohol, and fatty acid esters of polyoxyethylene sorbitan. One of ordinary skill in the art can easily determine with little or no experimentation the amount of sodium chloride, glucose, or glycerin necessary to make the injectable preparation isotonic. Additional ingredients, such as dissolving agents, buffer agents, and analgesic agents may be added. If necessary, coloring agents, preservatives, perfumes, seasoning agents, sweetening agents, and other medicines may also be added to the desired preparations during the treatment of schizophrenia.

The amount of amorphous ELT-HBr contained in a pharmaceutical composition according to the present invention is not specifically restricted; however, the dose should be sufficient to treat, ameliorate, or reduce the condition.

Having described the invention with reference to certain preferred embodiments, other embodiments will become apparent to one skilled in the art from consideration of the specification. The disclosures of the references referred to in this patent application are incorporated herein by reference. The invention is further defmed by reference to the following examples describing in detail the process and compositions of the invention. It will be apparent to those skilled in the art that many modifications, both to materials and methods, may be practiced without departing from the scope of the invention.

### EXAMPLES

### PXRD METHOD:

The PXRDs were taken with a Scintag X-Ray powder diffractometer model X'TRA, Cu-tube, solid state detector, where the sample holder was a round standard sample holder with round zero background plate with cavity of 25 (diameter)*0.5 (dept.) mm. The scanning parameters included a range of 2-40 degrees two-theta; a continuous scan mode with a step size of 0.05 deg. and a scan rate of 3 deg./min.

### LOD METHOD

The LOD (loss on drying) test was preformed with "Mettler toledo " LOD device under 80°C until stabilization of the weighed.

### Example 1: Preparation of Form β as reported in U.S. Patent No. 6,110,940

(a) 3-(N-Methyl-2(R)-pyrrolidinylmethyl)-5-(2-phenylsulphonylethyl)-1H-indole hydrobromide, β-Form

Hydrobromic acid (49% w/w, 27.86 ml, 0.25 mol) was added over 1 hour to a stirred solution of 3-(N-methyl-2(R)-pyrrolidinylmethyl)-5-(2-phenylsulphonylethyl)-1H-indole (92.86 g, 0.24 mol) in 1,2-dimethoxyethane (2.08 1) at about 5°C. The cooling bath was removed and the resulting slurry was allowed to granulate by stirring at room temperature for a further 18 hours. Filtration, followed by washing with 1,2-dimethoxyethane and drying *in vacuo*, afforded the required product (97.9 g) as a solid, m.p. 150-151°C. Found: C,56.77; H,5.87; N,5.85. C₂₂H₂₆N₂O₂S; HBr requires C,57.02; H,5.87; N,6.04%.

### Example 2: Preparation of Amorphous ELT-HBr

About 50 mg ELT-HBr Form β were slurried in 0.5 ml glycerol for 3 hours at room temperature. The solid material was isolated and the wet material was measured by PXRD. The PXRD confirmed that the wet material was amorphous ELT-HBr

### Example 3: Preparation of Amorphous ELT-HBr from ELT-HBr Monohydrate

Wet solid ELT-HBr monohydrate, (wet by acetone/water and obtained, for example, according to patent no. US 2005/0131237, example 4), was maintained at room temperature for 5 days in a hood; the solid obtained after drying was analyzed by PXRD and found to be amorphous ELT-HBr.

### Example 4: Preparation of Amorphous ELT-HBr by drying of ELT-HBr Monohydrate

Wet solid ELT-HBr monohydrate (obtained, for example, according to patent no. US 2005/0131237, example 4) was dried under about 50°C at about 20 mm Hg for 18 hours, and the solid obtained after drying was analyzed by PXRD and found to be amorphous ELT-HBr.

### Example 5: Preparation of Amorphous ELT-HBr from ELT-HBr Monohydrate

ELT-HBr monohydrate (50 mg), (obtained, for example, according to patent no. US 2005/0131237, example 4) was slurried in ethanol (0.5 ml) at room temperature and under magnetic stirring for 24 hours. The slurry was filtered and the wet material was analyzed by PXRD and found to be amorphous ELT-HBr.

### Example 6: Preparation of Amorphous ELT-HBr from ELT-HBr Monohydrate

ELT-HBr monohydrate, (obtained, for example, according to patent no. US 2005/0131237, example 4),(50 mg) was slurried in ethanol:ethyl acetate 80:20 (0.5 ml) at 80°C with magnetic stirring for 1.5 hours. Then it was cooled to room temperature and slurried for additional 16 hours. The slurry was filtered and the wet material was analyzed by PXRD and found to be amorphous ELT-HBr.

### Example 7: Preparation of Amorphous ELT-HBr from Form β

ELT-HBr Form β (45.83 mg) was slurried in THF:H₂0 7% (0.23 ml) at 80°C with magnetic stirring for 0.5 hours. Then it was cooled to 10°C and slurried for additional 3 hours. The slurry was filtered and the wet material was analyzed by PXRD and found to be amorphous ELT-HBr.

### Example 8: Preparation of Amorphous ELT-HBr

Example 7 was repeated using the following conditions: entries 1-5 suspend ELT-HBr Form β at the presented conditions. Entry 6 is a crystallization process of ELT-HBr at the presented conditions.

| | Weight (mg) | Solvent (ml) | Temp (°C)/cooling temp | Time (h)/cooling time | PXRD wet solid | PXRD dry solid |
|---|---|---|---|---|---|---|
| 1 | 51.7 | THF:H₂O 2% (0.26) | Room temperature | 3 | Amorphous | Monohydrate |
| 2 | 49.2 | THF:H₂O 2% (0.25) | 80/10 | 0.5/3 | Amorphous | Monohydrate +β |
| 3 | 46.4 | EtOAc :H₂O (0.23) | R.T. | 3 | Amorphous | Monohydrate +β |
| 4 | 58.2 | EtOAc :H₂O (0.3) | 80/10 | 0.5/3 | Amorphous | Monohydrate |
| 5 | 46.8 | EtOAc :H₂O (0.23) | 80/10 | 0.5/3 | Amorphous | β |
| 6 | 51.9 | MIBK (0.26) | 80/10 | 0.5/3 | Amorphous | β |

### Example 9: Preparation of ELT-HBr monohydrate from Amorphous ELT-HBr

The wet material obtained according to example 8, entries 1 and 4, was put in a vacuum oven, at a pressure of about 20 mm Hg and temperature of at about 60°C. The material obtained after about 16 hours was ELT-HBr monohydrate.

### Example 10: Preparation of a mixture of ELT-HBr monohydrate and ELT-HBr Form β from Amorphous ELT-HBr

The wet material obtained according to example 8, entries 2 and 3, was put in a vacuum oven, at a pressure of about 20 mm Hg and temperature of at about 60°C. The material obtained after about 16 hours was a mixture of ELT-HBr monohydrate and Form β.

### Example 11: Preparation of ELT-HBr form β from Amorphous ELT-HBr

The wet material obtained according to Example 8, entries 5-6, was put in vacuum oven at a pressure of about 20 mm Hg and temperature of at about 60°C for about 16 hours. The dry material was ELT-HBr form β.

### Example 12: Preparation of Amorphous ELT-HBr by crystallization of Form β

ELT-HBr Form β (48.6 mg) was slurried in EtOAc:H₂O 7% (0.24 ml) at 80°C, under magnetic stirring for 0.5 hour to obtain a clear solution. After cooling to 10°C, the solid precipitated and was slurried for another 3 hours at 10°C. The slurry was filtered and the wet material was analyzed by PXRD and found to be amorphous ELT-HBr.

### Example 13: Preparation of Amorphous ELT-HBr from ELT-HBr Form β

ELT-HBr Form β (55.44 mg) was slurried in ethylene glycol (0.28 ml) at 25°C under magnetic stirring for 2 hours to obtain a clear solution. After cooling to -19°C the solid precipitated. The slurry was filtered. The wet material was analyzed by PXRD and found to be amorphous ELT-HBr.

### Example 14: Preparation of Amorphous ELT-HBr by Spray Drying

A solution was prepared at room temperature with methanol (30 ml) and ELT-HBr (5 g). The solution was sprayed (440[ml/h]) to a chamber with hot nitrogen (35m³/h, 60°C) at co-current flow. The Atomizing flow (500[l/h]) of nitrogen gave the Droplets effect which lead to the high evaporation rate. The temperature of the outlet solids was 37-39° C.

### Example 15: Preparation of Amorphous ELT-HBr by Spray Drying

A solution was prepared at room temperature with methanol (30 ml) and ELT-HBr (5 g). The solution was sprayed (440[ml/h]) to the chamber with hot nitrogen (35m³/h, 120°C) at co-current flow. The Atomizing flow (500[l/h]) of nitrogen gave the Droplets effect which lead to the high evaporation rate. The temperature of the outlet solids was 73-75°C.

### Example 16: Preparation of ELT-HBr Form α from ELT-HBr Form β

ELT-HBr Form β (48.4 mg) was slurried in iso-butanol (0.48 ml) at 80°C under magnetic stirring for 3 hours. Then it was cooled to room temperature and slurried for another 0.5 hour. The slurry was filtered and the wet material was analyzed by PXRD and found to be ELT-HBr Form α.

### Example 17: Preparation of ELT-HBr Form α from ELT-HBr Form β

Example 16 was repeated using the following conditions:

| Weight (mg) | Solvent (ml) | Temp.(°C) | Time(h) | PXRD wet |
|---|---|---|---|---|
| 41.7 | Methyl acetate(0.42) | 80 | 23 | α |
| 41.2 | THF:H₂O 2%(0.41) | 55/25 | 3/0.5 | α |
| 46.2 | Cyclohexane(0.46) | 80/25 | 3/0.5 | α |

### Example 18: Preparation of ELT-HBr Form α from ELT-HBr Form β

ELT-HBr Form β (36.1 mg) was dissolved in EtOH (0.36 ml) at room temperature to obtain a clear solution. Then, by cooling to -19°C for 3 days, a precipitate was obtained which was isolated by filtration. The wet material was analyzed by PXRD and found to be ELT-HBr Form α.

### Example 19: Preparation of ELT-HBr Form α by drying ELT-HBr Form β

The wet solid ELT-HBr form β, (wet by normal butanol or by 1-propanol), was dried at 50°C and under vacuum for 23 hours. The solid obtained after drying was analyzed by PXRD and found to be a mixture of ELT-HBr Form β and ELT-HBr Form α.

### Example 20: Preparation of ELT-HBr Form β from Eletriptan base

Eletriptan base (11 g, ∼28.8 mmol) (prepared, for example, according to patent no. US 5, 545,644, example 32) was dissolved in IPA (226 ml) and neutralized by HBr/IPA (24 ml) to pH 7. The product precipitated as a light yellow syrup. The mother liquor was decanted, a fresh IPA (200 ml) was added and the mixture was triturated with spatula in an ultrasound bath (10 min). The mixture was then stored in a refrigerator overnight. The product was almost solid the next day. It was filtered off after 6 hours of stirring. The product was then washed with IPA and then by heptane and dried. Yield: 8.5 g, HPLC: 97.98% purity. The solid obtained after drying was analyzed by PXRD and found to be Form β.

### Example 21: Preparation of ELT-HBr Form β from Eletriptan base

Eletriptan base (7 g, 18.3 mmol) was dissolved in i-PrOH (150 ml) and neutralized by HBr/i-PrOH (0.67 M) to pH 7 - 7.5. ELT-HBr Form β seeds were added to the precipitating product obtained during the addition of HBr/i-PrOH and the mixture was then stirred for 6 hours. The white solid was filtered off washed with i-PrOH and then by heptane and dried under reduced pressure at 40°C for 5 hours. Yield: 5.56 g (66 %). The solid obtained after drying was analyzed by XRD and found to be ELT-HBr Form β.

### Example 22: Preparation of ELT-HBr Form β

A solution of HBr/i-PrOH (0.67 mol/l) was added to a stirred solution of purified eletriptan base (≥98 %) (9.5 g, 24.8 mmol) in i-PrOH (150 ml) at room temperature to until pH 7 was reached. After the addition of about 1/3 to 1/2 of the pre-calculated amount of HBr, the reaction was seeded by adding a small amount of ELT-HBr Form β. After the addition of HBr had been complete, the reaction mixture was stirred for 6 hours. The product was filtered off, washed with i-PrOH (40 ml) and then by heptane (40 ml) and dried under reduced pressure at 40°C. Yield: 8.05 g, 70%.

### Example 23: Preparation of ELT-HBr Form β as reported in U.S. Patent 6,380,226, Example 2, Step (a)

Hydrobromic acid (49% w/w, 27.86 ml, 0.25 mol was added over 1 hour to a stirred solution of 3N-methyl-2(R)-pyrrolidinylmethyl)-5-(2-phenylsulphonylethy)-1H-indole (92.86 g, 02 mol) in 1,2-dimethoxyethane (2.08 1) at about 5°C. The cooling bath was removed and the resulting slurry was allowed to granulate by stirring at room temperature for a further 18 hours. Filtration, followed by washing with 1,2-dimethoxyethane and drying in vacuo, afforded the required product (97.9 g) as a solid m.p. 150-151° C. Found C,56.77; H,5.87; N,5.85. C₂₂H₂₆N₂O₂S; HBr requires C,57.02; H,5.87; N,6.04%.

### Example 24: Preparation of ELT-HBr Form β

30 g of ELT-PTSA and 240 g of water were homogenized by stirring; 300 ml of MTBE was added and then NH₃ solution was added until pH of 10.5-11.0 was reached. A phase separation was preformed. 120 ml of 5% sodium carbonate was added to the organic phase, then stirring and another phase separation was preformed. The organic phase was distilled to dryness. 60 ml of IPA was then added and distillation to dryness was preformed again. Then 180 ml of IPA and 60 ml of acetone were added, the mixture was heated and solution of HBr in IPA was added until pH of 6.6 to 7.5 was reached, then seeding was preformed, the mixture was cooled and filtrated. The wet cake was dried under vacuum. The ELT-HBr was shown by XRD to be Form β.

### Example 25: Preparation of ELT-HBr Form β

30 g of ELT-PTSA and 240 g of water were homogenized by stirring; 300 ml of MTBE was added and then NH₃ solution was added until pH of 10.5-11.0 was reached. A phase separation was preformed. 120 ml of 5% sodium carbonate was added to the organic phase, then stirring and another phase separation was preformed. The organic phase was distilled to dryness. 60 ml of IPA was then added and distillation to dryness was preformed again. Then 240 ml of IPA were added, the mixture was heated and solution of HBr in IPA was added until pH of 6.6 to 7.5 was reached, then seeding was preformed, the mixture was cooled and filtrated. The wet cake was dried under vacuum. The ELT-HBr was shown by XRD to be Form β.

### Example 26: Preparation of ELT-HBr Monohydrate (dried) as reported in U.S. Patent Publication 2002/013358, Example 1

Eletriptan (2 kg) was dissolved in acetone (24.2 L) and filtered. The mixture was diluted with further acetone (7.41) and water (2.36 L) added. A chilled (<5°C) mixture of a solution of 48% by weight hydrogen bromide in water (0.863 kg) and acetone (12.4 L) was added in portions over about a 6 hour period whilst maintaining the temperature below 25°C throughout the addition. Full transfer of the hydrogen bromide solution was ensured by washing the residues into the reaction mixture using further acetone (2.4 L). The resulting slurry was granulated and chilled prior to collection of the product obtained by filtration. The product was washed carefully with acetone and then dried under reduced pressure and at ambient temperature in the presence of a water reservoir to provide ELT-HBr monohydrate (1.75 kg, 70%). This material was then milled before further use.

### Example 27: Preparation of ELT-HBr Monohydrate (wet) as reported in U.S. Patent Publication 2002/013358, Example 2

Eletriptan (1.9 kg) was dissolved in a solution of 97.5:2.5, by volume, THF:water (30 L) and filtered. A solution of hydrogen bromide (ca, 48% by weight) in water (0.87 kg) was added to the solution at 15-25°C. A dense crystalline slurry was formed. The slurry was heated under reflux for approximately one hour. The slurry was cooled to from 15 to 30° C. and granulated for a minimum of 1 hour. The product was filtered and washed with THF (10 L) to provide ELT-HBr monohydrate (23 kg). Analytical data obtained were identical to those obtained for the product of Example 1 (herein presented as Example 21).

### Example 28: Preparation of amorphous ELT-HBr

Eletriptan base solution (2100 ml IPA and 700 ml acetone - 8 volumes) was stirred at 42°C in a 10L glass reactor equipped with a glass impeller agitator and reflux condenser. Then, a HBr/IPA solution was slowly dropped to the solution until a final pH of 4.4 was attained. Spontaneous sedimentation occurred and seeding was took place. The pH was tested after 1 hr from the end of dropping and the value was rose to 6.5. Then, the temperature was kept at 40°C for additional 30 minutes, and then it was cooled to -10°C for 2 hours. After 1 hour at -10°C, the slurry was filtered and the wet material was washed with 350 ml of IPA. Then, the wet material was dried in a static vacuum tray oven at 40°C. Then, the dry material was analyzed by PXRD and found to be amorphous ELT-HBr.

### Example 29: Formulation of amorphous ELT-HBr

Solid pharmaceutical compositions of amorphous ELT-HBr and the following excipients: lactose monohydrate, sucrose and avicel were compacted into a dosage form like a tablet.

Further aspects and embodiments are set out in the following numbered clauses:
1. Amorphous eletriptan hydrobromide (ELT-HBr).
2. The amorphous ELT-HBr of clause 1 characterized by at least one of the PXRD patterns depicted in figures 1, 2, and 3.
3. The amorphous ELT-HBr of clause 1 having less than about 10% by weight of crystalline ELT-HBr.
4. The amorphous ELT-HBr of clause 3, having less than about 10% by weight of crystalline ELT-HBr is selected from the group consisting of: crystalline ELT-HBr characterized by PXRD pattern having peaks at 9.7, 10.7, 15.9, 16.5, 17.8, 18.3, 19.3, 19.8, 20.1, 21.2, 24.4, 25.5, 25.8, 26.7, 27.6, and 29.4 degrees 2θ, designated Form α ; crystalline ELT-HBr characterized by PXRD pattern having peaks at 11.0, 17.2, 19.2, 20.1, 21.6, 22.6, 23.6 and 24.8 degrees 2θ, designated as Form β; crystalline ELT-HBr characterized by PXRD pattern having peaks at 9.8, 12.5, 13.2, 13.6, 15.1, 16.2, 17.0, 17.5, 18.9, 19.7, 20.0, 21.9, 23.1, 23.6, 24.1, 27.4 and 29.7 degrees 20 designated monohydrate ELT-HBr; and mixtures thereof.
5. A process for preparing amorphous ELT-HBr comprising slurrying ELT-HBr Form β in a solvent selected from a group consisting of glycerol, mixtures of water and THF, and mixtures of ethylacetate and water wherein the mixture of water and THF contains at least about 2% to about 10% of water by volume.
6. The process of clause 5, wherein the solvent is a mixture of water and THF.
7. The process of clause 6, wherein the mixture contains about 5% to about 10% of water by volume.
8. The process of any of clauses 5 to 7, wherein the slurrying is done at a temperature of about room temperature to about 60°C.
9. The process of clause 5, wherein the solvent is a mixture of ethylacetate and water.
10. The process of clause 9, wherein the mixture is about 2% to 30% by volume water.
11. The process of any of clauses 9 to 10, wherein the slurrying is done at a temperature of about room temperature to about 80°C.
12. A process of preparing amorphous ELT-HBr comprising dissolving ELT-HBr in methylisobutyl ketone to form a solution, and precipitating amorphous ELT-HBr to obtain a suspension.
13. The process of clause 12, wherein the solution is provided by combining ELT-HBr and methylisobutyl ketone, and heating the combination.
14. The process of clause 12, wherein the precipitation of amorphous ELT-HBr occurs by cooling the solution to a temperature of about 10°C to obtain a suspension.
15. A process for preparing amorphous ELT-HBr comprising slurrying wet ELT-HBr monohydrate in a solvent selected from a group consisting of ethanol and mixtures of ethanol and ethylacetate.
16. The process of clause 15, wherein the solvent is ethanol.
17. The process of clause 16, wherein the slurrying is done at a temperature of about room temperature to the reflux temperature of the solvent.
18. The process of clause 15, wherein the solvent is a mixture of ethanol and ethylacetate.
19. The process of clause 18, wherein the ethanol is present in the solvent mixture in an amount of about 50% to 80% by volume.
20. The process of clause 18, wherein slurrying is done at a temperature of about room temperature to about 80°C.
21. A process of preparing amorphous ELT-HBr comprising providing a solution of ELT-HBr in mixtures of ethylacetate and water or in ethylene glycol, and precipitating amorphous ELT-HBr to obtain a suspension.
22. The process of clause 21, wherein the solvent is a mixture of ethylacetate and water.
23. The process of clause 21, wherein the solution of ELT-HBr in a mixture of ethylacetate and water is provided by heating a slurry of ELT-HBr in a mixture of solvents at a temperature of about 60°C to 80°C.
24. The process of clause 23, wherein the mixture of ethylacetate and water is about 2% to about 30% water.
25. The process of clause 21, wherein the solvent is ethylene glycol.
26. The process of clause 25, wherein the solution of ELT-HBr in ethylene glycol is provided by combining ELT-HBr and ethylene glycol at a temperature of about 0°C to about 25°C.
27. The process of clause 21, wherein the suspension of amorphous ELT-HBr is provided by cooling the solution at a temperature of about 10°C to about -20°C.
28. A process to prepare amorphous ELT-HBr comprising spray drying a solution of ELT-HBr in methanol.
29. A process for preparing amorphous ELT-HBr comprising heating wet monohydrate ELT-HBr at a temperature of at about room temperature to about 80°C and at atmospheric pressure to about 0.1 mm Hg.
30. Preparation of ELT-HBr Form α, ELT-HBr Form β or ELT-HBr monohydrate from amorphous ELT-HBr.
31. A process for preparing crystalline ELT-HBr Form α comprising slurrying ELT-HBr Form β in a solvent selected from a group consisting of isobutanol, methylacetate, mixtures of THF and water, and cyclohexane, wherein the mixture of water and THF contains from about 0.5.% of water by volume to about 2% of water by volume.
32. The process of clause 31 wherein the solvent is isobutanol, methylacetate, or cyclohexane.
33. The process of clause 31, wherein the slurrying is at a temperature of room temperature to about 80°C.
34. A process for preparing ELT-HBr Form α comprising crystallizing ELT-HBr from ethanol.
35. The process of clause 34, wherein the crystallization is done by a process comprising providing a solution of ELT-HBr in ethanol, and precipitating crystalline ELT-HBr Form α to obtain a suspension.
36. The process of clause 35, wherein the precipitation is done by cooling the solution at a temperature of about -5°C to about -20°C.
37. A process for preparing ELT-HBr Form α comprising heating wet crystalline ELT-HBr Form β.
38. The process of clause 37, wherein the heating is conducted at a temperature of about 45°C to about 60°C.
39. The process of clause 38, wherein the heating is conducted under reduced pressure of about 10 to about 30 mm Hg.
40. A process for preparing crystalline ELT-HBr Form β comprising, reacting eletriptan base and hydrobromic acid in isopropanol (IPA).
41. The process of clause 40, wherein the crystallization comprises
   a) combining eletriptan base with IPA to obtain a solution;
   b) combining the solution with hydrobromic acid to obtain a reaction mixture;
   c) precipitating ELT-HBr Form β, thus providing a suspension;
   d) separating the obtained eletriptan HBr from the reaction mixture;
   e) combining the separated eletriptan HBr with IPA providing a new mixture;
   f) placing the new mixture in an ultrasonic bath; and
   g) cooling the mixture of step f to a temperature of about 5°C to about 2°C providing the crystalline ELT-HBr Form β.
42. The process of clause 41, further comprising seeding of crystals of ELT-HBr Form β prior to step b.
43. The process of clause 41, wherein the pH of the reaction mixture in step b is from about 6 to about 8.
44. A process for preparing ELT-HBr Form β comprising maintaining wet amorphous ELT-HBr at a temperature of about 50°C to about 70°C.
45. A process for preparing ELT-HBr monohydrate comprising maintaining wet amorphous ELT-HBr at a temperature of about room temperature to about 60°C.
46. A pharmaceutical composition comprising of amorphous ELT-HBr and at least one pharmaceutically acceptable excipient.
47. A process for preparing the pharmaceutical composition of clause 46 comprising combining amorphous ELT-HBr and at least one pharmaceutically acceptable excipient.
48. A pharmaceutical composition comprising amorphous ELT-HBr made by the processes of any of the clauses 5, 12, 15, 21 28 and 29, and at least one pharmaceutically acceptable excipient.
49. A use of amorphous ELT-HBr for the manufacture of a pharmaceutical composition.
50. A use of amorphous ELT-HBr made by the processes of any of the clauses 5, 12, 15, 21 28 and 29 for the manufacture of a pharmaceutical composition.
51. A use of amorphous ELT-HBr for the manufacture of a medicament for the treatment of migraine headaches.
52. Use of amorphous ELT-HBr in a process for the manufacture of crystalline forms of eletriptan hydrobromide, preferably eletriptan crystalline Form α, ELT-HBr crystalline Form β or ELT-HBr monohydrate.

## Claims

1. A process for preparing eletriptan hydrobromide (ELT-HBr) Form β comprising combining eletriptan p-toluenesulfonic acid (ELT-PTSA) with water, adding methyl tert-butyl ether (MTBE) and NH₃ to cause a phase separation where after the organic phase is dried and combined with isopropanol (IPA) or mixtures of IPA and acetone, the pH is adjusted by the addition of HBr in IPA and the resulting mixture is cooled and the ELT-HBr Form β is collected.

2. A process according to Claim 1 comprising:
a) combining ELT-PTSA with water to form a first mixture;
b) adding MTBE to the first mixture;
c) adding NH₃ to the first mixture to cause the formation of a first organic phase and a first inorganic phase;
d) separating the first organic phase and first inorganic phase;
e) adding 5% sodium carbonate solution to the first organic phase to cause the formation of a second organic phase and a second inorganic phase;
f) separating the second organic phase and second inorganic phase;
g) drying the second organic phase to form a dried organic phase;
h) mixing the dried organic phase with isopropanol (IPA) or a mixture of IPA and acetone to form a second mixture;
i) adding HBr in IPA to the second mixture;
j) cooling the second mixture;
k) recovering the ELT-HBr Form β.

3. The process of Claim 2, wherein the pH of the first mixture after the addition of NH₃ is from about 10.5 to 11.0.

4. The process of any of Claims 2 to 3, further comprising adding IPA to the dried organic phase and drying prior to the step h).

5. The process of any of Claims 2 to 4, wherein the dried organic phase is mixed with a mixture of IPA and acetone.

6. The process of any of Claims 2 to 5 wherein the mixture in step h) is heated.

7. The process of Claim 5, wherein the mixture of IPA and acetone is up to a ratio of about 4:1 IPA:acetone by volume.

8. The process of any of Claims 2 to 7, wherein the pH of the second mixture after the addition of HBr is from about 6.6 to about 7.5.

9. The process of any of Claims 2 to 8, further comprising seeding the second mixture with ELT-HBr Form β prior to step j).
